# EUROPEAN PATENT SPECIFICATION

(11) **EP 4 110 319 B1**
(45) Date of publication and mention of the grant of the patent: **07.01.2026**
(21) Application number: 21759767.3
(22) Date of filing: 26.02.2021
(51) Int. Cl.: A61K 31/17, C12N 5/078, G01N 33/574, A61P 35/00

(54) **TUMOR-INFILTRATING LYMPHOCYTES WITH ENHANCED TUMOR REACTIVITY**
TUMORINFILTRIERENDE LYMPHOZYTEN MIT ERHÖHTER TUMORREAKTIVITÄT
LYMPHOCYTES INFILTRANT LES TUMEURS À RÉACTIVITÉ TUMORALE AMÉLIORÉE

(30) Priority: 27.02.2020 US 202062982524 P; 30.04.2020 US 202063017959 P; 27.01.2021 US 202163142153 P
(43) Date of publication of application: 04.01.2023
(73) Proprietor: H. Lee Moffitt Cancer Center & Research Institute, Inc., Tampa, Florida 33612 (US)
(72) Inventor: MULÉ, James J., Odessa, FL 33556 (US)
(74) Representative: Moré, Solveig Helga
(86) International application number: PCT/US2021/019985
(87) International publication number: WO 2021/174055

(56) References cited:
- WO-A1-2011/013129
- WO-A1-2011/013129
- WO-A2-2019/118873
- US-A1- 2019 284 640
- US-B2- 10 041 129
- HELENA HARLIN ET AL: "Chemokine expression in melanoma metastases associated with CD8+ T-cell recruitment", vol. 69, no. 7, 1 April 2009 (2009-04-01), pages 3077 - 3085, XP002670884, ISSN: 0008-5472, Retrieved from the Internet <URL:http://cancerres.aacrjournals.org/content/69/7/3077> [retrieved on 20090317], DOI: 10.1158/0008-5472.CAN-08-2281

## Description

### BACKGROUND

Melanoma is a serious form of skin cancer that begins in cells known as melanocytes. While it is less common than basal cell carcinoma (BCC) and squamous cell carcinoma (SCC), melanoma is far more dangerous because of its ability to spread to other organs more rapidly if it not treated at an early stage.

WO 2011/013129 A1 teaches the isolation and expansion of Tumor-infiltrating lymphocytes (TILs) that express at least one of the chemokine receptors selected from the group consisting of a CCL4 receptor, a CXCL1 receptor, a CXCL8 receptor, CXCR1 and a CXCL9 receptor. Harlin et al., 2009. Cancer Research, American Association of Cancer Research, US, 69(7):3077-3085 discloses that a subset of six chemokines (CCL2, CCL3, CCL4, CCL5, CXCL9, and CXCL10) are preferentially expressed in tumors that contained T cells.

### SUMMARY

Disclosed herein is a method of treating tumors in a subject that involves administering to the subject an effective amount of a composition comprising Tumor-infiltrating lymphocytes (TILs) with enhanced tumor reactivity produced from donor tumor cells, such as melanoma cells, that express a 12 Chemokine gene signature as defined below.

Also disclosed herein is a method for selecting TILs with enhanced tumor reactivity. These TILs are produced from donor melanoma cells with tertiary lymphoid structures (TLSs) that express the 12 Chemokine gene signature as defined below.

Thus, the invention provides tumor-infiltrating lymphocytes (TILs) with enhanced tumor reactivity for use in a method of treating tumors in a subject, wherein the TILs are produced from donor tumor cells with elevated gene expression of CCL2, CCL3, CCL4, CCL5, CCL8, CCL18, CCL19, CCL21, CXCL9, CXCL10, CXCL11, and CXCL13, wherein the tumor is a melanoma.

Also provided is a method for producing TILs with enhanced tumor reactivity, comprising
**a)** determining gene expression levels of CCL2, CCL3, CCL4, CCL5, CCL8, CCL18, CCL19, CCL21, CXCL9, CXCL10, CXCL11, and CXCL13 in tumor cells;
**b)** comparing the tumor gene expression levels to reference gene expression levels;
**c)** identifying tumor cells with gene expression levels above the reference gene expression levels; and
**d)** producing TILs from the tumor cells,
wherein the tumor is a melanoma.

The invention further provides a composition comprising TILs produced by the method of the invention.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 shows tertiary lymphoid structures (TLS) in melanoma identified by a 12 chemokine gene signature (12-CK GES).
FIG. 2 shows CD20 and CD3 expression in melanoma with 12-CK GES.
FIGs. 3A and 3B show response of a patient to TILs from melanoma with 12-CK GES.
FIG. 4 shows GES chemokines (CK) can enhance the production of melanoma peptide-reactive T cells.

### DETAILED DESCRIPTION

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

As will be apparent to those of skill in the art upon reading this disclosure, each of the individual embodiments described and illustrated herein has discrete components and features which may be readily separated from or combined with the features of any of the other several embodiments. Any recited method can be carried out in the order of events recited or in any other order that is logically possible.

Embodiments of the present disclosure will employ, unless otherwise indicated, techniques of chemistry, biology, and the like, which are within the skill of the art.

The following examples are put forth so as to provide those of ordinary skill in the art with a complete disclosure and description of how to perform the methods and use the probes disclosed and claimed herein. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary.

It must be noted that, as used in the specification and the appended claims, the singular forms "a," "an," and "the" include plural referents unless the context clearly dictates otherwise.

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

The term "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

The term "therapeutically effective" refers to the amount of the composition used is of sufficient quantity to ameliorate one or more causes or symptoms of a disease or disorder. Such amelioration only requires a reduction or alteration, not necessarily elimination.

The term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

### Chemokine gene signature

Disclosed herein are Tumor-infiltrating lymphocytes (TILs) with enhanced tumor reactivity produced from donor melanoma cells with tertiary lymphoid structures (TLSs) that express the herein disclosed chemokine gene signature.

Chemokines, which are small protein molecules involved in immune and inflammatory responses, direct leukocyte trafficking to areas of injury as well as to locations where primary immune responses are initiated (secondary lymphoid tissues such as lymph nodes, spleen, Peyer's patches, and tonsils). There are presently four classes of chemokine molecules (C, CC, CXC, and CX3C) that are named for the number and location of cysteine residues on the amino terminus of the protein. These molecules communicate with their target cells via G-protein coupled receptors that are pertussis toxin sensitive. Different chemokines act on different leukocyte populations, thereby modulating the influx of immune effector cells to the area in question based on the needs of the particular situation. Chemokines are secreted proteins involved in immunoregulatory and inflammatory processes. The chemokines of the disclosed gene signature are shown in Table 1.

| Table 1. Chemokines | | | |
|---|---|---|---|
| Gene Symbol | Gene Name | GenBank Acc. No: Nucleic Acid | GenBank Acc. No: Amino Acid |
| CCL2 | chemokine (C-C motif) ligand 2 | NM_002982.3 | NP_002973.1 |
| CCL3 | chemokine (C-C motif) ligand 3 | NM_002983.2 | NP_002974.1 |
| CCL4 | chemokine (C-C motif) ligand 4 | NM_002984.2 | NP_002975.1 |
| CCL5 | chemokine (C-C motif) ligand 5 | NM_002985.2 | NP_002976.2 |
| CCL8 | chemokine (C-C motif) ligand 8 | NM_005623.2 | NP_005614.2 |
| CCL18 | chemokine (C-C motif) ligand 18 (pulmonary and activation-regulated) | NM_002988.2 | NP_002979.1 |
| CCL19 | chemokine (C-C motif) ligand 19 | NM_006274.2 | NP_006265.1 |
| CCL21 | chemokine (C-C motif) ligand 21 | NM_002989.2 | NP_002980.1 |
| CXCL9 | chemokine (C-X-C motif) ligand 9 | NM_002416.1 | NP_002407.1 |
| CXCL10 | chemokine (C-X-C motif) ligand 10 | NM_001565.2 | NP_001556.2 |
| CXCL11 | chemokine (C-X-C motif) ligand 11 | NM_005409.4 | NP_005400.1 |
| CXCL13 | chemokine (C-X-C motif) ligand 13 | NM_006419.2 | NP_006410.1 |

The methods include assaying the presence or levels of chemokine mRNA or proteins in the sample. The presence and/or level of a protein can be evaluated using methods known in the art, e.g., using quantitative immunoassay methods. The presence and/or level of an mRNA can be evaluated using methods known in the art, e.g., Northern blotting or quantitative PCR methods, e.g., RT-PCR. In some embodiments, high throughput methods, e.g., protein or gene chips as are known in the art (see, e.g., Ch. 12, Genomics, in Griffiths et al., Eds. Modern genetic Analysis, 1999, W. H. Freeman and Company; Ekins and Chu, Trends in Biotechnology, 1999, 17:217-218; MacBeath and Schreiber, Science 2000, 289(5485):1760-1763; Simpson, Proteins and Proteomics: A Laboratory Manual, Cold Spring Harbor Laboratory Press; 2002; Hardiman, Microarrays Methods and Applications: Nuts & Bolts, DNA Press, 2003), can be used to detect the presence and/or level of chemokine proteins as described herein.

In some embodiments, the methods include assaying levels of one or more control genes or proteins, and comparing the level of expression of the chemokine genes or proteins to the level of the control genes or proteins, to normalize the levels of the chemokine genes or proteins. Suitable endogenous control genes includes a gene whose expression level should not differ between samples, such as a housekeeping or maintenance gene, e.g., 18S ribosomal RNA; beta Actin; Glyceraldehyde-3-phosphate dehydrogenase; Phosphoglycerate kinase 1; Peptidylprolyl isomerase A (cyclophilin A); Ribosomal protein L13a; large Ribosomal protein P0; Beta-2-microglobulin; Tyrosine 3-monooxygenase/tryptophan 5-monooxygenase activation protein, zeta polypeptide; Succinate dehydrogenase; Transferrin receptor (p90, CD71); Aminolevulinate, delta-, synthase 1; Glucuronidase, beta; Hydroxymethyl-bilane synthase; Hypoxanthine phosphoribosyltransferase 1; TATA box binding protein; and/or Tubulin, beta polypeptide.

Generally speaking, the methods described herein can be performed on cells from a tumor. The cells can be obtained by known methods, e.g., during a biopsy (such as a core needle biopsy), or during a surgical procedure to remove all or part of the tumor. The cells can be used fresh, frozen, fixed, and/or preserved, so long as the mRNA or protein that is to be assayed is maintained in a sufficiently intact state to allow accurate analysis.

In some embodiments of the methods described herein, the levels of the chemokine genes in the tumor sample can be compared individually to levels in a reference. The reference levels can represent levels in a melanoma that does not have tertiary lymphoid structures (TLSs). Alternatively, reference levels can represent levels in a melanoma that does have TLSs. In some embodiments, the reference levels represent a threshold.

In some embodiments of the methods described herein, values representing the levels of the chemokine genes can be summed to produce a "chemokine gene score" that can be compared to a reference chemokine gene score, wherein a chemokine gene score that is above the reference chemokine gene score indicates that the melanoma will produce TILs with enhanced tumor reactivity, and an chemokine gene score below the reference score indicates that the melanoma will produce TILs that do not have enhanced tumor reactivity.

For example, in some embodiments, the expression levels of each of the evaluated genes can be assigned a value (e.g., a value that represents the expression level of the gene, e.g., normalized to an endogenous control gene as described herein). That value (optionally weighted to increase or decrease its effect on the final score) can be summed to produce an immune-related gene score. One of skill in the art could optimize such a method to determine an optimal algorithm for determining an immune-related gene score.

The methods described herein include determining levels (or scores) for all of the 12 chemokines.

### Methods of Making TILs

Tumor-infiltrating lymphocyte (TIL) production is a 2-step process: 1) the pre-REP (Rapid Expansion) stage where fragments or single cell suspensions of fresh tumor are plated with interleukin-2 in standard lab media. The T cells that grow out in these pre-REP cultures are then selected, and 2) expanded to large numbers in the REP stage w/reagents such as irradiated feeder cells, and anti-CD3 antibodies to achieve the desired effect; i.e. to expand the TILs in a large enough culture amount for treating the patients. The REP stage requires cGMP grade reagents and 30-40 L of culture medium. However, the pre-REP stage can utilize lab grade reagents (under the assumption that the lab grade reagents get diluted out during the REP stage), making it easier to incorporate alternative strategies for improving TIL production.

Autologous TILs may be obtained from the parenchyma of resected fresh tumors. Tumor samples are obtained from patients and scalpel cut fragments or single cell suspension are made. The single cell suspension can be obtained in any suitable manner, e.g., mechanically (disaggregating the tumor using, e.g., a gentleMACS^{™} Dissociator, Miltenyi Biotec, Auburn, Calif.) or enzymatically (e.g., collagenase or DNase).

Expansion of lymphocytes, including tumor-infiltrating lymphocytes, such as T cells can be accomplished by any of a number of methods as are known in the art. For example, T cells can be rapidly expanded using non-specific T-cell receptor stimulation in the presence of feeder lymphocytes and interleukin-2 (IL-2), IL-7, IL-15, IL-21, or combinations thereof. The non-specific T-cell receptor stimulus can e.g. include around 30 ng/ml of OKT3, a mouse monoclonal anti-CD3 antibody (available from Ortho-McNeil^{®}, Raritan, N.J. or Miltenyi Biotec, Bergisch Gladbach, Germany).

Specific tumor reactivity of the expanded TILs can be tested by any method known in the art, e.g., by measuring cytokine release (e.g., interferon-gamma) or cytotoxicity following co-culture with tumor cells. In one embodiment, the cultured TILs are enriched for either CD8+ or CD4+ T cells prior to rapid expansion of the cells. Following culture of the TILs in IL-2, the T cells can be depleted of either CD4+ cells or CD8+ cells and enriched for CD4+ or CD8+ cells using, for example, a CD8/CD4 microbead separation (e.g., using a CliniMACS<plus >CD8/CD4 microbead system (Miltenyi Biotec)). In some embodiments, a T-cell growth factor that promotes the growth and activation of the autologous TILs are administered to the mammal either concomitantly with the autologous TIL or subsequently to the autologous TIL. The T-cell growth factor can be any suitable growth factor that promotes the growth and activation of the autologous TIL. Examples of suitable T-cell growth factors include interleukin (IL)-2, IL-7, IL-15, IL-12 and IL-21, which can be used alone or in various combinations, such as IL-2 and IL-7, IL-2 and IL-15, IL-7 and IL-15, IL-2, IL-7 and IL-15, IL-12 and IL-7, IL- 12 and IL-15, or IL-12 and IL2. IL-12 is a preferred T-cell growth factor.

### Therapeutic Uses

The invention provides tumor-infiltrating lymphocytes (TILs) with enhanced tumor reactivity for use in a method of treating tumors in a subject, wherein the TILs are produced from donor tumor cells with elevated gene expression of CCL2, CCL3, CCL4, CCL5, CCL8, CCL18, CCL19, CCL21, CXCL9, CXCL10, CXCL11, and CXCL13, wherein the tumor is a melanoma.

TILs can elicit an anti-tumor immune response against tumor cells wherein the tumor is a melanoma. This anti-tumor immune response elicited by the disclosed TILs may be an active or a passive immune response. In addition, the TILs may be part of an adoptive immunotherapy approach in which TILs induce an immune response.

The disclosed TILs may be administered either alone, or as a pharmaceutical composition in combination with diluents and/or with other components such as IL-2, IL-15, or other cytokines or cell populations. Briefly, pharmaceutical compositions may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions for use in the disclosed methods are in some embodiments formulated for intravenous administration. Pharmaceutical compositions may be administered in any manner appropriate treat the cancer. The quantity and frequency of administration will be determined by such factors as the condition of the patient, and the severity of the patient's disease, although appropriate dosages may be determined by clinical trials.

When "an immunologically effective amount", "an anti-tumor effective amount", "an tumor-inhibiting effective amount", or "therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the TILs described herein may be administered at a dosage of 10⁴ to 10⁹ cells/kg body weight, such as 10⁵ to 10⁶ cells/kg body weight, including all integer values within those ranges. TIL compositions may also be administered multiple times at these dosages. The cells can be administered by using infusion techniques that are commonly known in immunotherapy (see, e.g., Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly.

The administration of the disclosed compositions may be carried out in any convenient manner, including by injection, transfusion, or implantation. The compositions described herein may be administered to a patient subcutaneously, intradermally, intratumorally, intranodally, intramedullary, intramuscularly, by intravenous (i.v.) injection, or intraperitoneally. In some embodiments, the disclosed compositions are administered to a patient by intradermal or subcutaneous injection. In some embodiments, the disclosed compositions are administered by i.v. injection. The compositions may also be injected directly into a tumor, lymph node, or site of infection.

In certain embodiments, the disclosed TILs are administered to a patient in conjunction with (e.g., before, simultaneously or following) any number of relevant treatment modalities, including but not limited to thalidomide, dexamethasone, bortezomib, and lenalidomide. In further embodiments, the TILs may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, cytoxin, fludaribine, cyclosporin, FK506, rapamycin, mycophenolic acid, steroids, FR901228, cytokines, and irradiation. In some embodiments, the TILs are administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In another embodiment, the cell compositions of the present invention are administered following B-cell ablative therapy such as agents that react with CD20, e.g., Rituxan. For example, in some embodiments, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

In some aspects, the melanoma can be any neoplasm or tumor for which radiotherapy is currently used. Alternatively, the melanoma can be a neoplasm or tumor that is not sufficiently sensitive to radiotherapy using standard methods.

The disclosed TILs can be used in combination with any compound, moiety or group which has a cytotoxic or cytostatic effect. Drug moieties include chemotherapeutic agents, which may function as microtubulin inhibitors, mitosis inhibitors, topoisomerase inhibitors, or DNA intercalators, and particularly those which are used for cancer therapy.

The disclosed TILs can be used in combination with a checkpoint inhibitor. The two known inhibitory checkpoint pathways involve signaling through the cytotoxic T-lymphocyte antigen-4 (CTLA-4) and programmed-death 1 (PD-1) receptors. These proteins are members of the CD28-B7 family of cosignaling molecules that play important roles throughout all stages of T cell function. The PD-1 receptor (also known as CD279) is expressed on the surface of activated T cells. Its ligands, PD-L1 (B7-H1; CD274) and PD-L2 (B7-DC; CD273), are expressed on the surface of APCs such as dendritic cells or macrophages. PD-L1 is the predominant ligand, while PD-L2 has a much more restricted expression pattern. When the ligands bind to PD-1, an inhibitory signal is transmitted into the T cell, which reduces cytokine production and suppresses T-cell proliferation. Checkpoint inhibitors include, but are not limited to antibodies that block PD-1 (Nivolumab (BMS-936558 or MDX1106), CT-011, MK-3475), PD-L1 (MDX-1105 (BMS-936559), MPDL3280A, MSB0010718C), PD-L2 (rHIgM12B7), CTLA-4 (Ipilimumab (MDX-010), Tremelimumab (CP-675,206)), IDO, B7-H3 (MGA271), B7-H4, TIM3, LAG-3 (BMS-986016).

Human monoclonal antibodies to programmed death 1 (PD-1) and methods for treating cancer using anti-PD-1 antibodies alone or in combination with other immunotherapeutics are described in U.S. Patent No. 8,008,449. Anti-PD-L1 antibodies and uses therefor are described in U.S. Patent No. 8,552,154. Anticancer agent comprising anti-PD-1 antibody or anti-PD-L1 antibody are described in U.S. Patent No. 8,617,546.

In some embodiments, the PDL1 inhibitor comprises an antibody that specifically binds PDL1, such as BMS-936559 (Bristol-Myers Squibb) or MPDL3280A (Roche). In some embodiments, the PD1 inhibitor comprises an antibody that specifically binds PD1, such as lambrolizumab (Merck), nivolumab (Bristol-Myers Squibb), or MEDI4736 (AstraZeneca). Human monoclonal antibodies to PD-1 and methods for treating cancer using anti-PD-1 antibodies alone or in combination with other immunotherapeutics are described in U.S. Patent No. 8,008,449. Anti-PD-L1 antibodies and uses therefor are described in U.S. Patent No. 8,552,154. Anticancer agent comprising anti-PD-1 antibody or anti-PD-L1 antibody are described in U.S. Patent No. 8,617,546.

The disclosed TILs can be used in combination with other cancer immunotherapies. There are two distinct types of immunotherapy: passive immunotherapy uses components of the immune system to direct targeted cytotoxic activity against cancer cells, without necessarily initiating an immune response in the patient, while active immunotherapy actively triggers an endogenous immune response. Passive strategies include the use of the monoclonal antibodies (mAbs) produced by B cells in response to a specific antigen. The development of hybridoma technology in the 1970s and the identification of tumor-specific antigens permitted the pharmaceutical development of mAbs that could specifically target tumor cells for destruction by the immune system. Thus far, mAbs have been the biggest success story for immunotherapy; the top three best-selling anticancer drugs in 2012 were mAbs. Among them is rituximab (Rituxan, Genentech), which binds to the CD20 protein that is highly expressed on the surface of B cell malignancies such as non-Hodgkin's lymphoma (NHL). Rituximab is approved by the FDA for the treatment of NHL and chronic lymphocytic leukemia (CLL) in combination with chemotherapy. Another important mAb is trastuzumab (Herceptin; Genentech), which revolutionized the treatment of HER2 (human epidermal growth factor receptor 2)-positive breast cancer by targeting the expression of HER2.

Generating optimal "killer" CD8 TIL responses may also require T cell receptor activation plus co-stimulation, which can be provided through ligation of tumor necrosis factor receptor family members, including OX40 (CD134) and 4-1BB (CD137). OX40 is of particular interest as treatment with an activating (agonist) anti-OX40 mAb augments T cell differentiation and cytolytic function leading to enhanced anti-tumor immunity against a variety of tumors.

In some embodiments, such an additional therapeutic agent may be selected from an antimetabolite, such as methotrexate, 6-mercaptopurine, 6-thioguanine, cytarabine, fludarabine, 5-fluorouracil, decarbazine, hydroxyurea, asparaginase, gemcitabine or cladribine.

In some embodiments, such an additional therapeutic agent may be selected from an alkylating agent, such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin.

In some embodiments, such an additional therapeutic agent may be selected from an anti-mitotic agent, such as taxanes, for instance docetaxel, and paclitaxel, and vinca alkaloids, for instance vindesine, vincristine, vinblastine, and vinorelbine.

In some embodiments, such an additional therapeutic agent may be selected from a topoisomerase inhibitor, such as topotecan or irinotecan, or a cytostatic drug, such as etoposide and teniposide.

In some embodiments, such an additional therapeutic agent may be selected from a growth factor inhibitor, such as an inhibitor of ErbBI (EGFR) (such as an EGFR antibody, e.g. zalutumumab, cetuximab, panitumumab or nimotuzumab or other EGFR inhibitors, such as gefitinib or erlotinib), another inhibitor of ErbB2 (HER2/neu) (such as a HER2 antibody, e.g. trastuzumab, trastuzumab-DM I or pertuzumab) or an inhibitor of both EGFR and HER2, such as lapatinib).

In some embodiments, such an additional therapeutic agent may be selected from a tyrosine kinase inhibitor, such as imatinib (Glivec, Gleevec STI571) or lapatinib.

Therefore, in some embodiments, a disclosed antibody is used in combination with ofatumumab, zanolimumab, daratumumab, ranibizumab, nimotuzumab, panitumumab, hu806, daclizumab (Zenapax), basiliximab (Simulect), infliximab (Remicade), adalimumab (Humira), natalizumab (Tysabri), omalizumab (Xolair), efalizumab (Raptiva), and/or rituximab.

In some embodiments, a therapeutic agent for use in combination with TILs for treating the disorders as described above may be an anti-cancer cytokine, chemokine, or combination thereof. Examples of suitable cytokines and growth factors include IFNy, IL-2, IL-4, IL-6, IL-7, IL-10, IL-12, IL-13, IL-15, IL-18, IL-23, IL-24, IL-27, IL-28a, IL-28b, IL-29, KGF, IFNa (e.g., INFa2b), IFN , GM-CSF, CD40L, FIt3 ligand, stem cell factor, ancestim, and TNFa. Suitable chemokines may include Glu-Leu-Arg (ELR)- negative chemokines such as IP-10, MCP-3, MIG, and SDF-la from the human CXC and C-C chemokine families. Suitable cytokines include cytokine derivatives, cytokine variants, cytokine fragments, and cytokine fusion proteins.

In some embodiments, a therapeutic agent for use in combination with a TILs for treating cancers as described above may be a cell cycle control/apoptosis regulator (or "regulating agent"). A cell cycle control/apoptosis regulator may include molecules that target and modulate cell cycle control/apoptosis regulators such as (i) cdc-25 (such as NSC 663284), (ii) cyclin-dependent kinases that overstimulate the cell cycle (such as flavopiridol (L868275, HMR1275), 7-hydroxystaurosporine (UCN-01, KW-2401), and roscovitine (R-roscovitine, CYC202)), and (iii) telomerase modulators (such as BIBR1532, SOT-095, GRN163 and compositions described in for instance US 6,440,735 and US 6,713,055) . Non-limiting examples of molecules that interfere with apoptotic pathways include TNF-related apoptosis-inducing ligand (TRAIL)/apoptosis-2 ligand (Apo-2L), antibodies that activate TRAIL receptors, IFNs, and anti-sense Bcl-2.

In some embodiments, a therapeutic agent for use in combination with TILs for treating cancers as described above may be a hormonal regulating agent, such as agents useful for anti-androgen and anti-estrogen therapy. Examples of such hormonal regulating agents are tamoxifen, idoxifene, fulvestrant, droloxifene, toremifene, raloxifene, diethylstilbestrol, ethinyl estradiol/estinyl, an antiandrogene (such as flutaminde/eulexin), a progestin (such as such as hydroxyprogesterone caproate, medroxy- progesterone/provera, megestrol acepate/megace), an adrenocorticosteroid (such as hydrocortisone, prednisone), luteinizing hormone-releasing hormone (and analogs thereof and other LHRH agonists such as buserelin and goserelin), an aromatase inhibitor (such as anastrazole/arimidex, aminoglutethimide/cytraden, exemestane) or a hormone inhibitor (such as octreotide/sandostatin).

In some embodiments, a therapeutic agent for use in combination with TILs for treating the cancers as described above may be an anti-cancer nucleic acid or an anti-cancer inhibitory RNA molecule.

Combined administration, as described above, may be simultaneous, separate, or sequential. For simultaneous administration the agents may be administered as one composition or as separate compositions, as appropriate.

In some embodiments, the disclosed TILs are administered in combination with radiotherapy. Radiotherapy may comprise radiation or associated administration of radiopharmaceuticals to a patient is provided. The source of radiation may be either external or internal to the patient being treated (radiation treatment may, for example, be in the form of external beam radiation therapy (EBRT) or brachytherapy (BT)). Radioactive elements that may be used in practicing such methods include, e.g., radium, cesium-137, iridium-192, americium-241, gold-198, cobalt-57, copper-67, technetium-99, iodide-123, iodide-131, and indium-111.

In some embodiments, the disclosed TILs are administered in combination with surgery.

### EXAMPLES

### Example 1:

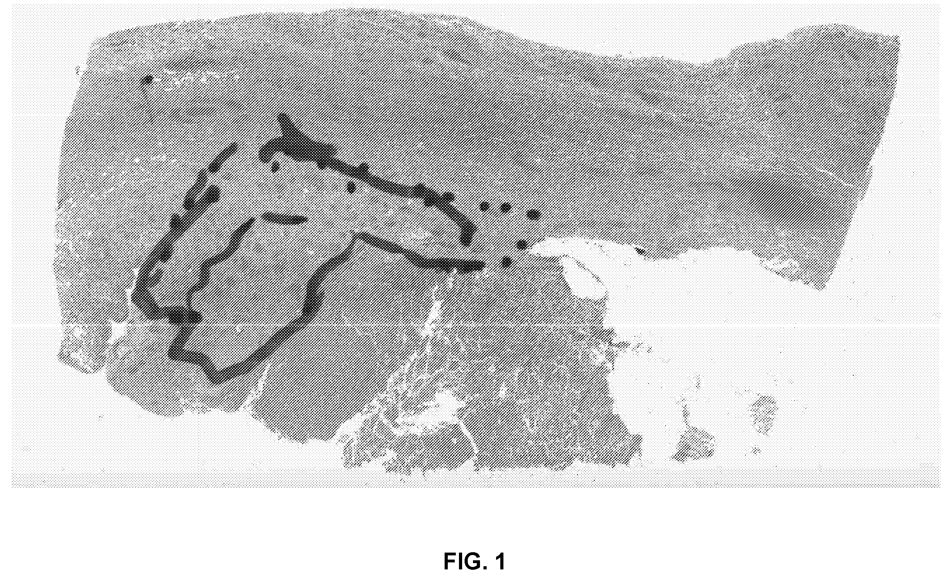

FIG. 1 shows tertiary lymphoid structures (TLS) in melanoma identified by a 12 chemokine gene signature (12-CK GES).

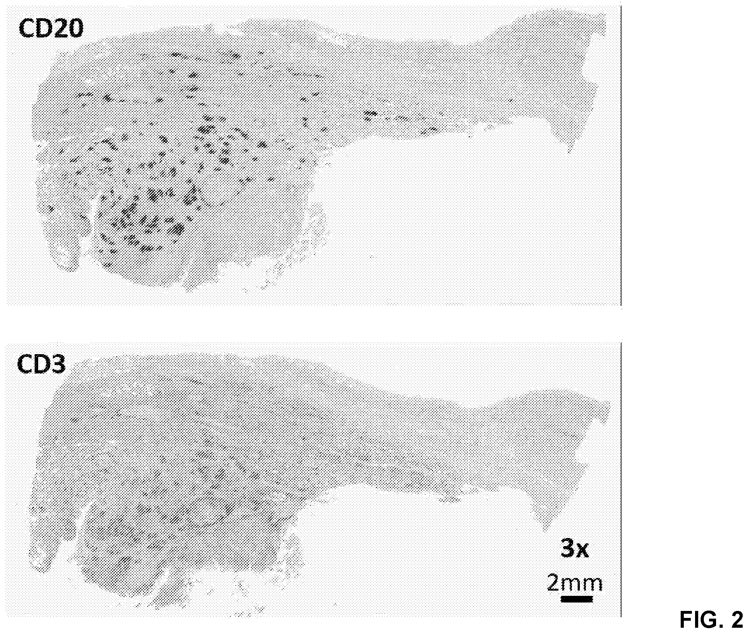

FIG. 2 shows CD20 and CD3 expression in melanoma with 12-CK GES.

| Table 2. Melanomas with Tertiary Lymphoid Structures Generate Higher Frequencies of TIL with Anti-tumor Reactivity* | | | | | | |
|---|---|---|---|---|---|---|
| | # fragments | | | # fragments | | |
| | plated | TIL + | | tested | IFN-gamma + | |
| Control* | 425 | 130 | 31% | 130 | 50 | 38% |
| TLSs* | 126 | 102 | 81% | 102 | 96 | 94% |
| * A retrospective analysis | | | | | | |
| **Fragments from unselected melanomas | | | | | | |
| ***Fragments from melanomas selected for 12-CK GES positivity, including Patient #8 | | | | | | |

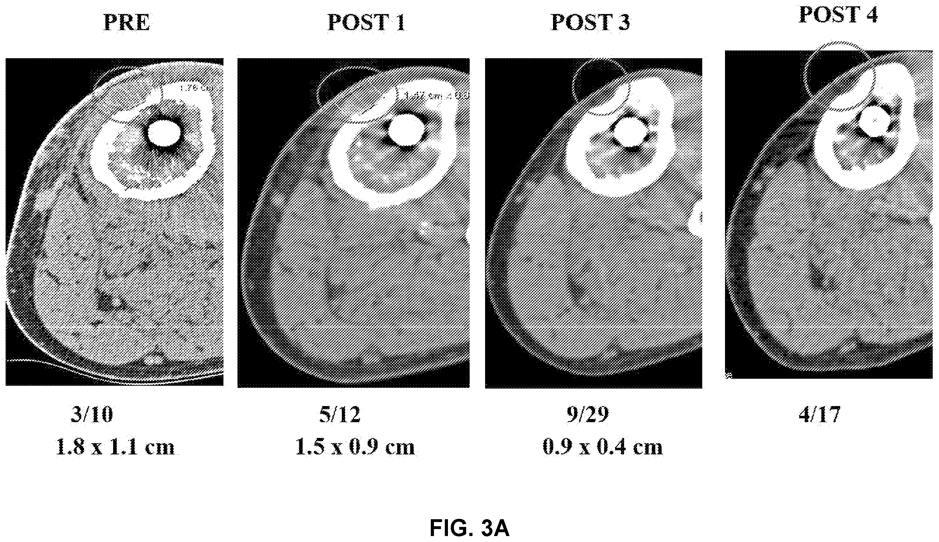

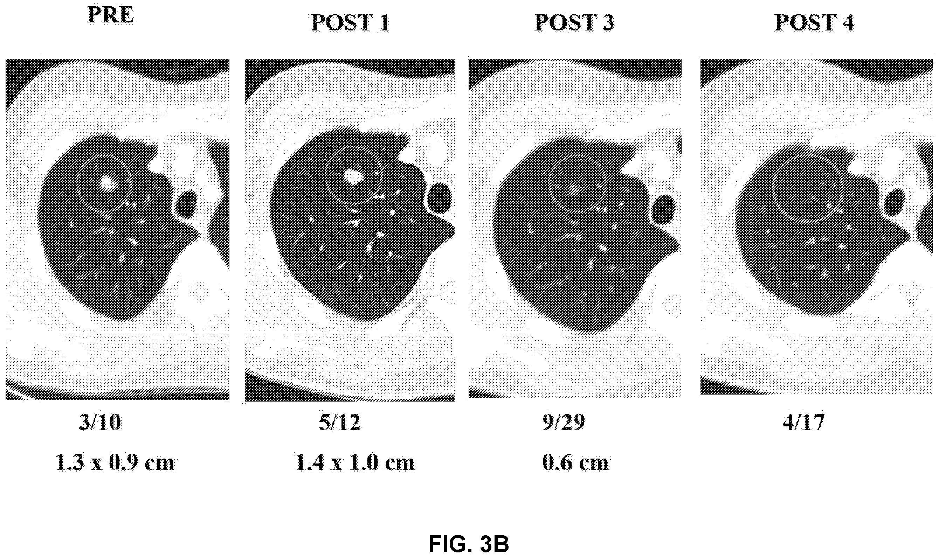

FIGs. 3A and 3B show response of a patient to TILs from melanoma with TLSs.

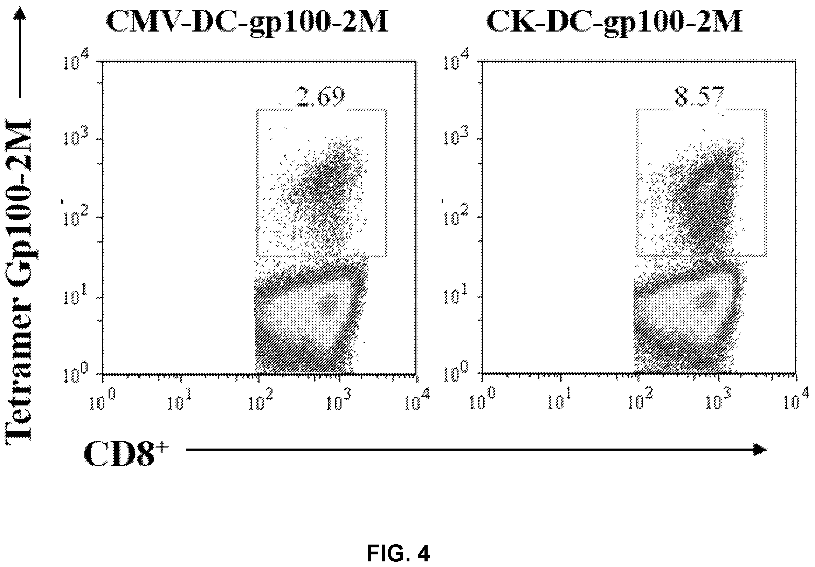

FIG. 4 shows GES chemokines (CK) can enhance the production of melanoma peptide-reactive T cells.

## Claims

1. Tumor-infiltrating lymphocytes (TILs) with enhanced tumor reactivity for use in a method of treating tumors in a subject, wherein the TILs are produced from donor tumor cells with elevated gene expression of CCL2, CCL3, CCL4, CCL5, CCL8, CCL18, CCL19, CCL21, CXCL9, CXCL10, CXCL11, and CXCL13, wherein the tumor is a melanoma.

2. A method for producing TILs with enhanced tumor reactivity, comprising
(a) determining gene expression levels of CCL2, CCL3, CCL4, CCL5, CCL8, CCL18, CCL19, CCL21, CXCL9, CXCL10, CXCL11, and CXCL13 in tumor cells;
(b) comparing the tumor gene expression levels to reference gene expression levels;
(c) identifying tumor cells with gene expression levels above the reference gene expression levels; and
(d) producing TILs from the tumor cells,
wherein the tumor is a melanoma.

3. A composition comprising TILs produced by the method of claim 2.

## Patentansprüche

1. Tumorinfiltrierende Lymphozyten (TILs) mit erhöhter Tumorreaktivität zur Verwendung in einem Verfahren zur Behandlung von Tumoren in einem Subjekt, wobei die TILs aus Spendertumorzellen mit erhöhter Genexpression von CCL2, CCL3, CCL4, CCL5, CCL8, CCL18, CCL19, CCL21, CXCL9, CXCL10, CXCL11 und CXCL13 hergestellt werden, wobei der Tumor ein Melanom ist.

2. Ein Verfahren zur Herstellung von TILs mit erhöhter Tumorreaktivität, umfassend
(a) Bestimmung der Genexpressionsniveaus von CCL2, CCL3, CCL4, CCL5, CCL8, CCL18, CCL19, CCL21, CXCL9, CXCL10, CXCL11 und CXCL13 in Tumorzellen;
(b) Vergleich der Tumorgenexpressionsniveaus mit Referenzgenexpressionsniveaus;
(c) Identifizierung von Tumorzellen mit Genexpressionsniveaus oberhalb der Referenzgenexpressionsniveaus; und
(d) Herstellung von TILs aus den Tumorzellen,
wobei der Tumor ein Melanom ist.

3. Eine Zusammensetzung, die TILs, die durch das Verfahren nach Anspruch 2 hergestellt wurden, umfasst.

## Revendications

1. Lymphocytes infiltrant les tumeurs (TIL) à réactivité tumorale améliorée pour une utilisation dans une méthode de traitement de tumeurs chez un sujet, dans lesquels les TIL sont produits à partir de cellules tumorales donneuses ayant une expression génique supérieure de CCL2, CCL3, CCL4, CCL5, CCL8, CCL18, CCL19, CCL21, CXCL9, CXCL10, CXCL11 et CXCL13, dans lesquels la tumeur est un mélanome.

2. Méthode de production de TIL à réactivité tumorale améliorée, comprenant les étapes de
(a) déterminer les niveaux d'expression génique de CCL2, CCL3, CCL4, CCL5, CCL8, CCL18, CCL19, CCL21, CXCL9, CXCL10, CXCL11 et CXCL13 dans les cellules tumorales;
(b) comparer les niveaux d'expression génique tumoraux à des niveaux d'expression génique de référence;
(c) identifier des cellules tumorales ayant des niveaux d'expression génique supérieurs aux niveaux d'expression génique de référence ; et
(d) produire des TIL à partir des cellules tumorales,
dans laquelle la tumeur est un mélanome.

3. Composition comprenant des TIL produits par le procédé selon la revendication 2.
